Europäisches Patentamt

European Patent Office

Office européen des brevets

(19)

(11) Publication number: **0 157 638**
A2

(12) **EUROPEAN PATENT APPLICATION**

(21) Application number: 85302324.0

(22) Date of filing: 02.04.85

(51) Int. Cl.⁴: **C 12 P 19/14, C 12 N 9/28**

(30) Priority: 03.04.84 US 596275

(43) Date of publication of application: 09.10.85
Bulletin 85/41

(84) Designated Contracting States: **AT BE CH DE FR GB IT LI LU NL SE**

(71) Applicant: **NOVO INDUSTRI A/S, Novo Allé, DK-2880 Bagsvaerd (DK)**

(72) Inventor: **Hansen, Tomas Tage, Plantagevej 18, DK-3460 Birkerod (DK)**
Inventor: **Rugh, Susanne, Sophienbergvej 25, DK-2960 Rungsted Kyst (DK)**

(74) Representative: **Brown, John David et al, FORRESTER & BOEHMERT Widenmayerstrasse 4/I, D-8000 München 22 (DE)**

(54) **Method for production of high conversion syrups and immobilized alpha-amylase employed in the process.**

(57) A method for production of high conversion syrup wherein a liquefied starch is hydrolyzed with an $\alpha$-amylase covalently immobilized inside a carrier substance.

Use of the novel immobilized $\alpha$-amylase provides a single step reaction wherein the liquefied starch solution is converted into a high conversion syrup through the action of a single enzyme. Preferred $\alpha$-amylases are acid stable $\alpha$-amylases derived from Aspergillus and a well suited acid stable $\alpha$-amylase is derived from Aspergillus niger strain DSM 2761.

0157638

This invention relates to a method for production of high conversion syrups and to the immobilized α-amylases employed in the process.

BACKGROUND OF THE INVENTION

High conversion syrups are characteristically elevated in glucose and maltose. Their uses, which largely are in confectionary (soft candy), for canning, soft-drinks, brewing and baking dictate that the syrups will not crystallize at above 4°C and 80 - 83% DS (dry substance). The general composition ranges for high conversion syrups are DE 60 - 70, glucose not exceeding 39% (DS), maltose 25 - 37% (DS), balance $DP_3$ and higher. More rigid specifications sometimes applied are: DE 62 - 65, glucose 35% ± 3% (DS), maltose 30% ± 2% (DS), $DP_3$ 13% ± 3% (DS), $DP_{4+}$ 22% ± 3% (DS).

High conversion syrups have heretofore been made by enzymatic treatment of liquefied starch, usually of acid liquefied starch and by a combination of maltogenic amylases and glucoamylase. The process is hard to control and is cumbersome. A large scale practice attainment of the more rigid specifications for high conversion syrup are rather difficult.

The principal object of this invention is to provide a simplified process for forming high conversion syrups from liquefied starch solutions.

RATIONALE OF THE INVENTION

The simplest possible process for forming high conversion syrups would, of course, be a single step reaction or reactor wherein the liquefied starch solution converts into a high conversion syrup through the action of a suitable enzyme or enzymes. Thus in analogy with the widely used batch method of saccharifying liquified starch to glucose syrups it can be hoped that a liquid form maltogenic

enzyme would catalyze conversion of a liquefied starch solution batch into a high conversion syrup.

Unfortunately the commercially available α-amylases are wrong for the processes, producing starch hydrolysis spectra that do not correspond to high conversion syrups neither when used in economical nor in process allowable amounts.

Aspergillus niger and other black Aspergilli species produce an acid-stable α-amylase that is thermostable and productive of an interesting starch hydrolysate spectrum, particularly when glucoamylase also has been added to the liquified starch.

However, soluble form use of the acid stable α-amylase by itself does not produce high conversion syrups. Also soluble form use of bacterial neutral α-amylases e.g. from Bacillus licheniformis and Bacillus subtilis such as those sold under the Trade Marks Termamyl© and BAN©, respectively are not suitable for industrial application.

Surprisingly, it has now been discovered that acid-stable α-amylases *and neutral α-amylases* alone catalyze conversion of starch hydrolysates into high conversion syrups provided the α-amylases are present in an immobilized form.

BRIEF STATEMENT OF THE INVENTION

Briefly stated the process of this invention comprises hydrolyzing a liquefied starch solution with an α-amylase covalently immobilized inside a carrier substance, as for example in the form of enzyme containing granules. Such forms of the α-amylases are well suited to employment as the packed bed inside of a continuous operation column reactor.

The hydrolysis is conducted at a temperature in the range of 55 - 70°C and at a pH of 3 - 7.

As the hydrolysis is conducted at a relatively high temperature the α-amylases must be sufficiently thermostable under the conditions to perform the hydrolysis.

The pH is preferably 3 - 5 and the DE of the liquified starch is preferably 12 - 55, more preferably 30 - 55.

According to a preferred embodiment of the present invention the α-amylase in the carrier is immobilized from an α-amylase preparation of an acid-stable Aspergillus derivable α-amylase which preparation is essentially free of transferase and glucoamylase activity. The acid-stable α-amylases are preferably derivable from Aspergillus niger and most preferably the acid stable α-amylase is derivable from Aspergillus niger strain DSM 2761.

Further preferred α-amylases are neutral α-amylases from Bacillus, e.g. from Bacillus subtilis and Bacillus licheniformis.

The present invention also provides an immobilized α-amylase product comprising an α-amylase covalently immobilized inside of a carrier substance for preparing high conversion syrups according to the claimed method.


DISCUSSION OF THE INVENTION


The Enzymes
Acid-stable α-amylases

The acid-stable enzyme produced by more than one Aspergillus species of the A. niger group can be employed to hydrolyze liquefied starch solution into high conversion syrups.

Acid-stable alpha-amylases are known from Canadian patent 663,274 and several articles in Agr.Biol.Chem. (Y.Minoda et al., Agr.Biol.Chem. vol. 27, No. 11, p. 806 - 811, 1963, vol. 32, No. 1, p. 104 - 109, 1968 and vol. 32, No. 1, p. 110 - 113, 1968).

The acid-stable alpha-amylase may be produced as described in the prior art by cultivating different black Aspergillus species, e.g. A. niger, A. usamii and A.awamori under acid conditions at first (pH 2.5 - 4.5) and then under slight acid conditions (pH about 5.5), or else as described hereinafter for a preferred embodiment. In addition to the acid-stable alpha-amylase also an acid-unstable-alpha-

amylase (neutral amylase), and enzymes with glucoamylase and transferase activities are formed by the wild strains.

Insofar as the inventors hereof can ascertain variations in the acid-stable α-amylase A. niger group species to A. niger group species are immaterial within the context of this invention, which causes them to expect that an acid-stable α-amylase made of A. species other than those reported in the literature to produce the enzyme will generate essentially the same acid-stable α-amylase.

The acid-stable α-amylase product as elaborated by the wild strains of the Aspergillus is, unfortunately, not suited to practice of this invention, due to presence of undesirable side activities, notably glucoamylase activity and transferase activity. For practice of this invention the acid-stable Aspergillus α-amylase product should be essentially free of transferase and of glucoamylase activity, and such an acid-stable α-amylase product could of course be made according to the teachings in the above noted Canadian patent and literature citations by fractional precipitation with ammonium sulphate, rivanol and acetone whereby the acid-stable α-amylase in crystalline form is obtained.

The acid-stable Aspergillus α-amylase preparation preferred for practice of this invention is elaborated essentially free of transferase and glucoamylase activity by an A. niger strain that has been mutated so as not to elaborate the undesired activities. Further discussion of the acid-stable α-amylase will be in reference to practice of the invention with this preferred mode of acid-stable α-amylase.

In a preferred embodiment of the present invention a strain of Aspergillus niger has been mutated to block the synthesis of enzymes with amyloglucosidase and transferase activities whilst retaining fully the ability to synthesize the acid-stable alpha-amylase. A culture of such a mutant strain (TSA-1) of Aspergillus niger has been deposited with The German Collection of Microorganisms (DSM), Göttingen,

Germany, on 2nd September, 1983 and has been assigned the reference number DSM 2761.

## Neutral α-amylases

Further suitable α-amylases for the practice of the present invention are neutral α-amylases from Bacillus subtilis and Bacillus licheniformis (W.M. Fogarty, "Microbial Enzymes and Biotechnology" Applied Science, London 1983, pp. 1 - 92, Eds: W.M. Fogarty) e.g. the commercially available thermostable Bacillus subtilis α-amylase such as that sold under the Trade Mark BAN® and the commercially available thermostable Bacillus licheniformis α-amylase such as that sold under the Trade Mark TERMAMYL®, both supplied by NOVO Industri A/S, Denmark.

Other α-amylases may be used according to the present invention, e.g. from Bacillus stearothermophilus, provided that they are sufficiently thermostable to be used in the hydrolyzing process at high temperature.

## DETERMINATION OF ENZYME ACTIVITY

### Acid-stable α-amylase

One acid-stable α-amylase unit (FAU) is defined as the amount of soluble enzyme which under standard conditions (temperature 37°C, pH 4.7 (0.01 M acetate buffer) and reaction time 1 hour) hydrolyses 5.26 g starch (dry substance) (Merck) so that the hydrolyzed starch is only slightly coloured by addition of iodine-iodine potassium (4.04 g/liter KJ and 0.022 g/liter J ) and standardizing by comparing with an enzyme standard using a Daylite Comparator Illuminator.

Acid-stable alpha-amylase can be determined after inactivating of neutral amylase if present by acid incubation with 0.1 M HCl at pH 2.5. After incubation at 37°C for 30 minutes pH is adjusted to 4.7 with NaOH. The acid-stable alpha-amylase is obtained in 100% yield by this procedure.

Neutral α-amylases

One kilo NOVO neutral α-amylase unit (KNU) is defined as the amount of enzyme which under standard conditions (temperature 37°C, pH 5.6, 0.0003 M $Ca^{2+}$) hydrolyses 5.26 g starch (dry substance) per hour so that the solution is only slightly-coloured by iodine as described above.

PREPARATION OF THE ACID-STABLE α-AMYLASE

The preparation of an acid-stable alpha-amylase will now be described in detail:

Spores of Aspergillus niger mutant TSA-1 are transferred from an agar slab to sterile water and transferred aseptically to an inoculation tank containing a suitable fermentation medium containing assimilable sources of carbon and nitrogen and inorganic salts. Stirring and aeration is started and fermentation is carried out at about 30 - 36°C until there is good growth in the tank (or for about 48 hours).

Part of the tank content is transferred to a main fermentation tank (2000 liter) containing a suitable fermentation medium containing assimilable carbon and nitrogen sources and inorganic salts. pH is about 5.0 - 6.5 and aerating and stirring is started. Fermentation is carried out at 30 - 36°C and pH is adjusted to about 3.2 - 5.0 after about 24 hours fermentation. The total fermentation time is about 100 - 200 hours.

The culture liquid is then freed from fungus mycelium by filtration and centrifugation. If desired the liquid may be concentrated by evaporation and preserving agents may be added.

Typically, the resulting enzyme concentrate exhibits an activity in the range of about 50 to 300 FAU/ml.

The enzyme concentrate is essentially without maltase activity (no glucose formation was found after 70

hours' incubation at 70°C of about 25% DS maltose with 0.32
FAU of enzyme per g maltose).

## Preparation of other α-amylases

The preparation of neutral α-amylases derived from
Bacillus subtilis and Bacillus licheniformis are well known
in the art. If a commercial  preparation is not available,
a concentrate can be produced as described above for the
acid-stable α-amlylases.

## ENZYME CHEMICAL PROPERTIES

## Acid-stable α-amylase

The temperature and pH dependence of the activity
of the acid-stable alpha-amylase concentrate prepared as
described in Example 1 was determined by the method for
determination of acid-stable α-amylase activity described
above, using a reaction mixture of which the temperature and
pH were adjusted to predetermined values. Reference is now
made to the attached drawing in which

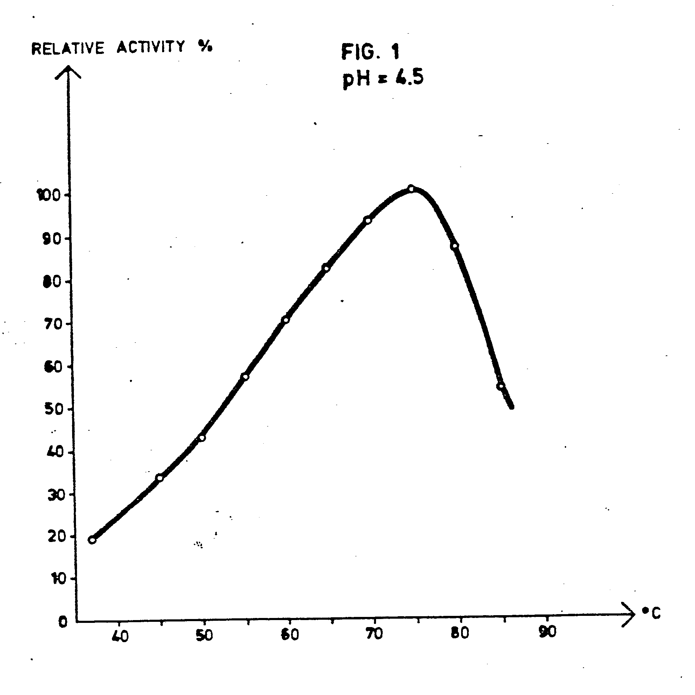

Fig. 1 graphically illustrates the relative activity plotted against temperature at pH 4.5, and

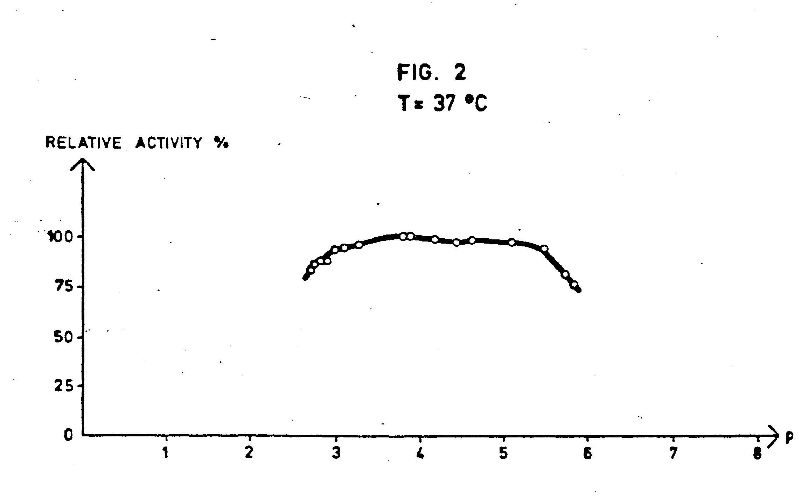

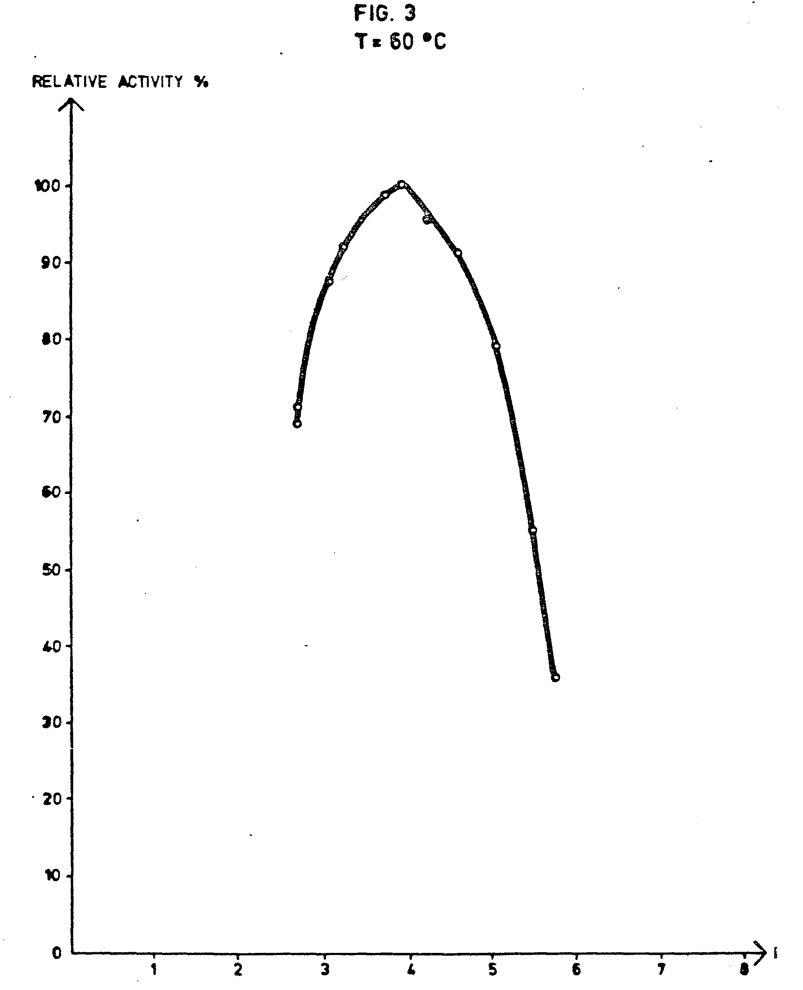

Fig. 2 and 3 graphically illustrate the relative
activity plotted against pH (adjusted with acetate
and citrate buffer) at 37°C and 60°C, respectively.

It appears that the enzyme concentrate has an activity
optimum of about 75°C at pH 4.5 and that its pH optimum is
in the range of from 3 - 5.

The thermostability of the acid-stable alpha-
amylase prepared as described in Example 1 was determined
after addition of 100 mg of $Ca^{++}$ per liter concentrate and
adjusting pH to 5.0. The residual amylase activity was
determined by the method described above. The results appear
from the following table:

Table I

| Temperature °C | Per cent residual activity after 30 min. |
|---|---|
| 70 | 100 |
| 75 | 82 |
| 80 | 10 |

Neutral α-amylase

The enzyme chemical properties of α-almylases from Bacillus subtilis and Bacillus licheniformis are well known, see W.M. Fogarty, ibid.

Notwithstanding removal by purification or through mutation of the production strain of transferase and glucoamylase activity, soluble form use of the acid-stable Aspergillus α-amylase by itself does not produce high conversion syrups as earlier described. Also, soluble forms neutral α-amylases from Bacillus licheniformis and Bacillus subtilis such as those sold under the Trade Marks Thermamyl® and BAN®, respectively do not produce the high conversion syrups.

It has now been found that surprisingly immobilized forms of the above α-amylases are capable of catalyzing a direct formation of high conversion syrups. Described below are typical experimental results obtained with the different α-amylases.

Illustrated in table II is the striking difference between the hydrolysis spectrum of the soluble acid-stable α-amylase and the immobilized enzyme.

The substrate was a 40 - 42 DE acid liquefied starch syrup from Roquette Freres. The pH of the syrup was adjusted to 4.5 and the dry substance content was adjusted to 45%. The experiment was performed at 60°C. The dosage level was 0.16 FAU/g DS for the soluble enzyme and 0.16 IFAU/g DS (as hereinafter defined) for the immobilized enzyme in the batch experiments. The batch saccharification time was 24 hours.

Table II

| | %DP$_1$ | %DP$_2$ | %DP$_3$ | %DP$_{4+}$ | DE |
|---|---|---|---|---|---|
| Acid liquefied 41 DE syrup | 16 | 13 | 12 | 59 | 41 |
| High-conversion syrup | 35±3 | 30±2 | 13±3 | 22±3 | 62 - 65 |
| Solub. enzyme | 19 | 33 | 25 | 22 | 58 |
| Glucoamylase added | 36 | 35 | 9 | 21 | 66 |
| Immobil. enzyme, batch experiment | 32 | 34 | 12 | 22 | 64 |
| Immobil. enzyme column experiment | 33 | 32 | 13 | 22 | 64 |

Also, the neutral α-almylases show a difference between the hydrolysis spectrum of the soluble enzyme and the immobilized enzyme (see example 12).

Mention already has been made that the immobilized α-amylases are inside a carrier substance, such not being the case when an enzyme is immobilized by adsorption or by covalent bonding to the surface of a carrier substance (see Hyashida et al., Agric.Biol. Chem. 46 (6) 1639 - 1645, (1982)). The immobilization technique per se forms no part of this invention, and virtually any of the many enzyme immobilization methods suggested to the art for covalently bonding enzymes in a carrier substance are believed to be suitable for forming the immobilized enzyme of this invention. The immobilization methods described in DE-patent application No P 3336257.2 and DE-patent application No. P 3336235.1 are somewhat preferred.

It is believed by the inventors hereof that the reason for striking DP$_1$, DP$_3$ differences in the hydrolysis spectrum from soluble enzyme compared to immobilized enzyme (see the above table II) can be attributed to the microenvironment present inside the soaked carrier substance. The higher molecular weight dextrins, the DP$_{4+}$ fraction notably, which diffuse into the carrier are sterically retained therein, offering the enzyme immobilized inside the carrier substance opportunity to hydrolyze the $DP_{4+}$ molecules within the particles to the limit dextrins, which mostly are $DP_1$ and $DP_2$ saccharides. Absence of transferase activity and of glucoamylase activity in the enzyme is then of major significance.

## CHEMICAL PROPERTIES OF IMMOBILIZED α-AMYLASES
### Immobilized acid-stable α-amylase

The temperature and pH dependence of the activity of the immobilized acid-stable α-amylase was determined by the method for determination of immobilized α-amylase activity described below using an acid liquefied syrup of which the temperature and pH were adjusted to predetermined values. The immobilized enzyme in a dose of 14 IFAU was added to 15 ml of a 42 DE acid liquefied syrup at 45% DS, buffered with 0.01 M acetate buffer and kept in a thermostated shaking water bath for two hours. The final syrup was analysed and the DE-value was taken as an expression of activity.

Reference is now made to the attached drawing in which

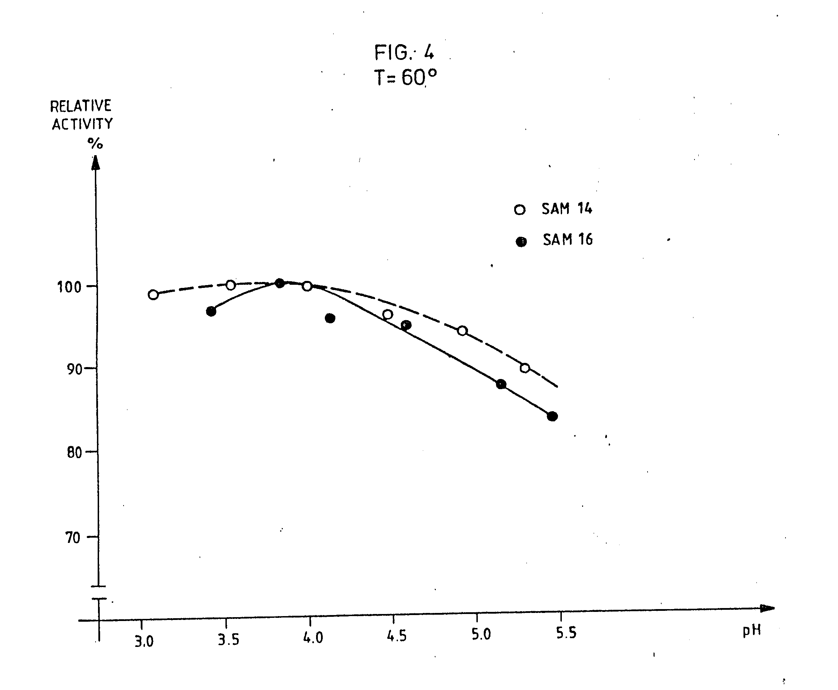

Fig. 4 graphically illustrates the relative activity plotted against pH at 60°C for the two different immobilized preparations described in examples 3 and 5, and

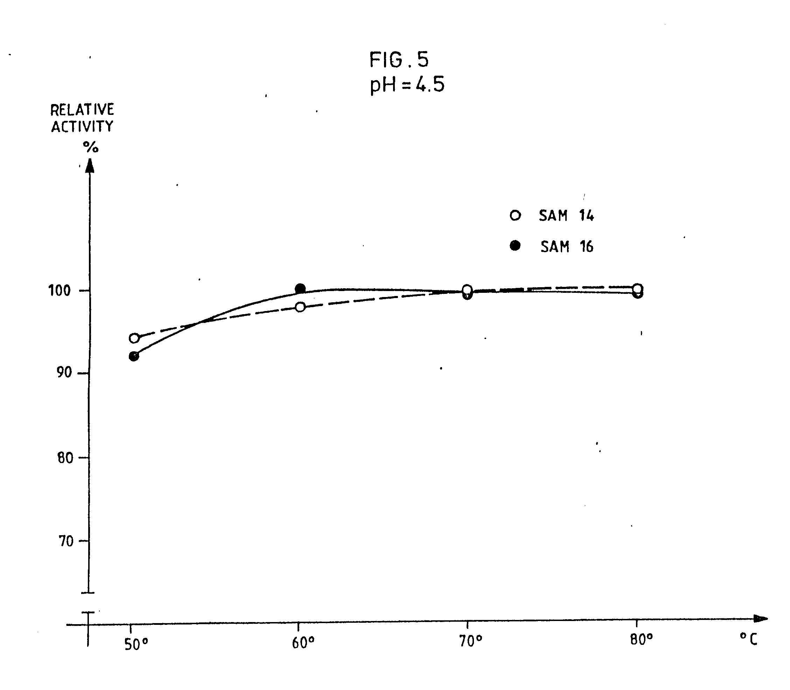

Fig. 5 graphically illustrates the relative activity plotted against temperature at pH 4.5 for the two different immobilized preparations described in examples 3 and 5.

It appears that the immobilized enzyme has an activity optimum in the range of 70 - 80°C at pH 4.5 and that its pH optimum is in the range of 3.5 - 4.5.

## DETERMINATION OF IMMOBILIZED ENZYME ACTIVITY
### Immobilized acid-stable α-amylase

One IFAU is defined as the amount of immobilized acid-stable α-amylase preparation which gives the same decline

in $DP_{4+}$ from a dextrin substrate as 1 FAU soluble *acid stable* ~~A. niger~~
α-amylase when measured under the conditions stated below.

Analysis Conditions for Immobilized ~~A. niger~~ *acid stable* α-amylase

| | |
|---|---|
| Substrate: | 30% w/w maltodextrin MD 03 L, CPC |
| Temperature: | 60°C |
| pH: | 4.5 |
| Reaction time: | 1 hour in a water bath shaker. |

The reaction is stopped by filtering off the immobilized
enzyme particles.
The reaction with soluble enzyme is stopped by changing pH
to about 9 and boiling.

Immobilized neutral α-amylase

One IKNU is defined as the amount of immobilized
neutral α-amylase preparation which gives the same decline
in $DP_{4+}$ from a dextrin substrate as 1 KNU soluble neutral
α-amylase when measured under the conditions stated below.

Analysis Conditions for Immobilized Neutral α-amylase

| | |
|---|---|
| Substrate: | 30% w/w maltodextrin MD 03 L, CPC |
| Temperature: | 60°C |
| pH: | 4.5 |
| Reaction time: | 1 hour in a water bath shaker. |

The reaction is stopped by filtering off the immobilized
enzyme particles.
The reaction with soluble enzyme is stopped by changing pH
to about 9 and boiling.
The carbohydrate spectra are determined by HPLC.

THE SUBSTRATE

Contemplated for practice of this invention thereon are liquefied starches preferably in the DE range of 12 -
55, a more preferred DE range being 30 - 55. The reasonably elevated DE 30 level ensures viscosity and diffusion characteristics for the syrups which allows the dextrin molecules to penetrate the carrier substance. On the other hand the DE 55 upper limit is basically an arbitrary limit which may be too high or too low and would depend upon the nature of the syrup. It so happens that the random spectrum of acid hydrolyzed starch, DE about 40 for example is most convenient for practice of this invention, and acid hydrolyzed starch is a preferred liquefied starch substrate for practice of this invention. Employment of enzymatically liquefied starch is, of course, contemplated.

PROCESS PARAMETERS

Temperature:

The operating temperature to be chosen is a compromise between high activity, good stability and the risk of microbial contamination. As seen in Fig. 5 the activity increases over the temperature range 50 - 70°C, but on the other hand the half-life is decreased when the temperature is increased. The risk of microbial contamination is lowest at the high temperature. We have found that 60°C gives a good productivity and low risk of microbial contamination. In industrial scale use the temperature giving the lowest cost of conversion and a microbially safe process is most likely to be within the claimed range of 55°C to 70°C and may vary from plant to plant.

pH:

From fig. 4 it is seen that a good operating range for acid-stable α-amylases is pH 3 - 5, a more preferred range being 4 - 5 because the enzyme stability has been found to be greater in the higher part of the pH range.

For neutral α-amylases a good operating range is pH 4.5 - 7 and a more preferred range is pH 5.5 - 6.5.

Substrate dry substance

The α-amylases are stabilized by high substrate content so the high dry substance 30 - 50% DS, normally used for enzyme or acid liquefaction processes is well suited for the saccharification process with immobilized α-amylases.

For further understanding of the invention the following examples are provided.

Example 1

Preparation of acid stable α-amylase

In an inoculation tank a volume of 300 liter of the following medium was prepared:

| | |
|---|---|
| Corn steep liquor: | 7.2 kg |
| Glucose: | 7.2 kg |
| $CaCO_3$: | 0.9 kg |
| Pluronic L 61: | 30 ml |
| Water: | up till 300 liter |

pH was adjusted to 5.5 before addition of $CaCO_3$ and sterilization. The mixture was inoculated with spores of strain DSM 2761 from a Fernbach flask containing C-1 agar (3% sucrose, 0.1% $KH_2PO_4$, 0.3% $NaNO_3$, 0.5% $MgSO_4$,$7H_2O$, 0.01% $FeSO_4$, $7H_2O$, 0.05% KCl and 2.5% agar in demineralized water) which had been incubated for about 7 days at 30°C. Stirring (300 rpm) and aeration (300 Nl/min) was started and fermentation was carried out at 34°C for about 51 hours.

150 liter of the tank content were then transferred to a main fermentation tank containing:

| | |
|---|---|
| Glucose: | 340 kg |
| Soy bean meal: | 85 kg |
| $KH_2PO_4$: | 5.1 kg |
| $Na_2HPO_4$,$12H_2O$ : | 6.8 kg |
| Pluronic L61: | 150 ml |
| Water: | up till 1700 liter |

During the first 18 hours of fermentation aeration was slowly increased from 500 to 1700 Nl/min and stirring was increased from 0 to 250 rpm. The fermentation was carried out at 34°C. pH was 6.2 before inoculation and was after fermentation for 24 hours slowly lowered to 3.2 - 3.5 by addition of phosphorous acid. pH was kept at 3.2 - 3.5 during the rest of the fermentation. Total fermentation time was 140 - 160 hours.

The culture liquid containing 1.3 FAU/ml was pretreated with 3% Clarit BW 100 (Bentonit) at pH 4.0 and drum filtrated on a precoat vacuum drum filter with HSC as precoat (HSC kieselguhr from John Manville). The drum filtrate was filtrated on Seitz plates supra 100 and then ultra/diafiltrated on Romicon PM-10 membranes.

After ultrafiltration the concentrate was filtrated on a frame filter press (Schule) with HSC and CBL/3 (kieselguhr from CECA) as filter aid. Finally the filtrate was evaporated until 38% RD (refractometer dry solid) and preserved with sodiumbenzoate (0.1%) and potassium sorbat (0.1%).

The obtained enzyme concentrate had an enzyme activity of 67 FAU/ml. The content of sugar alcohols was 1.3% by weight and the concentrate had no maltase activity.

After incubation for 30 min. at pH 2.5 and 37°C a residual acid-stable amylase activity of 100% was found indicating that no neutral alpha-amylase was produced during the above fermentation process.


Example 2

Saccharification test with soluble acid stable α-amylase

A 42 DE acid liquefied syrup was used as substrate and diluted with deionized water corresponding to a dry substance content of 45%. pH was adjusted to 4.5 and enzyme was added in a dosage corresponding to 0.16 FAU/g dry solid. The flasks were placed in a water bath at 60°C during stirring. Aliquots of 25 ml were taken after 0, 2, 24, and 48 hours and analysed on HPLC. The results are tabulated below.

Table III

```
Reaction time,
hours        %DP_1  %DP_2  %DP_3  %DP_4+  DE

  0            18     11     11     59     42

  2            19     17     19     45     46

 24            22     33     24     21     56

 48            25     36     20     19     58
```

Example 3

Partly purified A. niger acid-stable α-amylase, prepared as described in Example 1, was further purified by the following procedure.

To 1 kg enzyme containing solution was added 3 kg water and 200 g Clarcel CBL 3 as filter-aid. pH was adjusted to 4.5. The mixture was filtered on a pressurized laboratory filter. The precipitate was discarded.

1434 g $(NH_4)_2SO_4$ was added to the filtrate. After 1.5 hours at 4°C the mixture was centrifuged at 3500 rpm for 30 minutes. The supernatant was discarded and the precipitate was dissolved in 795 ml 0.05 M acetate buffer pH 4.5. The redissolved precipitate was then dialyzed against ion-exchanged water and concentrated on an Amicon hollow fiber dialyzer.

The concentrate contained 14% DS and the A. niger acid-stable α-amylase activity was 115 FAU/g (determined according to the method described above)

Part of the enzyme concentrate was further concentrated in vacuum from 14% DS to 22.5% DS. 12.5 g of the enzyme solution with 22.5% DS was combined with 15 g dry carrier particles prepared as described in DE-patent application No. P 3336235.1 example 6.

The enzyme solution was exchanged with the air in the carrier particles by vacuumsuction for 1 hour.

The enzyme containing particles were transferred to 375 ml of a solution containing 22% $Na_2SO_4$, 2% sodium acetate and 0.15% active glutaraldehyde, pH = 6.0. By this

procedure the <u>A</u>. <u>niger</u> α-amylase was crosslinked to the carrier particles.

After 4 hours the particles were washed 5 times with 100 ml 3% sodium acetate solution at pH 4.5.

The moist particles were frozen and filled into a column after thawing. This product is identified as SAM-10.

Also SAM-1, SAM-2 and SAM-14 were prepared as described here, only the glutaraldehyde concentration used was 0.1% in preparation of SAM-1 and 0.2% in preparation of SAM-2 and SAM-14.

## Example 4

35.7 g of the enzyme concentrate with 14% DS (described in example 3) was diluted with ion-exchanged water to 8% DS. pH was adjusted to 6.0 and 1.5 ml 50% glutaraldehyde solution was added in order to crosslink the enzyme. After 1 hour, the crosslinked enzyme is flocculated out of the solution with 1.5 ml Superfloc C521. The flocculate is filtered off on a laboratory filter whereafter excess water is squeezed out of the flocculate by hand. The moist filtercake is granulated on a 1 mm sieve and dried at room temperature over night. The activity was 14 IFAU/g. This product is identified as SAM-3.

## Example 5

47.8 g of the enzyme concentrate with 14% DS (as described in example 3) was mixed with 7 g albumen dissolved in 30 ml water with 0.6 g $CaCl_2$, $2H_2O$.

The pH was adjusted to 6.0 with 4 N NaOH. About 5 minutes after addition of 2.5 ml 50% glutaraldehyde solution the mass gelled to a firm gel. The gel was granulated on a 1 mm sieve and washed with 100 ml water. Excess water was drained off and the granules were dried at room temperature over night. The activity was determined to 12.6 IFAU/g by the method described above.

This product was identified as SAM-5. SAM-4, SAM-11 and SAM-16 were prepared by the same method.

Example 6

As a preliminary test 4 different immobilized forms of A. niger acid-stable α-amylase were used to saccharify a 42 DE acid ~~liquefied~~ *liquefied* syrup under batch conditions. The preparations were SAM-1 and SAM-2 (described in example 3), SAM-3 (described in example 4) and SAM-4 (described in example 5). The temperature was 60°C. The pH was 4.5 and DS was 45%. A sample was taken after 24 hours and 48 hours for HPLC and DE analysis. The results are tabulated below.

Table IV

| | 24 hours | | | | | 48 hours | | | | |
| Sample No | %DP$_1$ | %DP$_2$ | %DP$_3$ | %DP$_{4+}$ | DE | %DP$_1$ | %DP$_2$ | %DP$_3$ | %DP$_{4+}$ | DE |
|---|---|---|---|---|---|---|---|---|---|---|
| SAM-1 | 29 | 35 | 16 | 21 | 62 | 38 | 35 | 9 | 18 | 67 |
| SAM-2 | 28 | 34 | 16 | 22 | 61 | 37 | 34 | 10 | 18 | 67 |
| SAM-3 | 32 | 34 | 12 | 22 | 62 | 43 | 32 | 6 | 18 | 71 |
| SAM-4 | 25 | 34 | 16 | 25 | 57 | 33 | 36 | 10 | 21 | 64 |
| substrate | 18 | 11 | 11 | 59 | 42 | | | | | |

Example 7

8.8 g of SAM-5 dry particles were soaked in a 42 DE acid liquefied starch syrup for two hours. The size of the dry particles was within the range of 300 - 850 μm. Beforehand pH of the syrup was adjusted to 4.5 and the dry substance content reduced from about 80 DS to 45%. To avoid bacterial contamination, 100 ppm Kathon 886 was added to the syrup. The particles were filled into a water jacketed column with a diameter of 1.5 cm. The column was maintained at 60°C and the acid liquefied starch syrup was continuously pumped through the enzyme bed. The flow rate was controlled in order to keep the product syrup at a DE of about 62. Using the DE-value as an expression of the activity of the enzyme, the halflife of the enzyme was estimated to approximately 1500 hours. The initial activity of the particles was found to be 2.5 g product/h/g particles all calculated on dry basis.

Composition of the syrups.

| | %DP$_1$ | %DP$_2$ | %DP$_3$ | %DP$_{4+}$ | DE |
|--------|------|------|------|------|----|
| Inlet  | 16 | 13 | 12 | 59 | 42 |
| Outlet | 32 | 32 | 14 | 22 | 62 |

The outlet pH was 4.3 - 4.4 and the column was run for more than 2000 hours.

Example 8.

8.5 g dry particles of SAM-11 were soaked and filled in a column as described in example 7. The size distribution of the dry particles was

| Particle size | Amount of particles |
|---------------|---------------------|
| > 1000 µm     | 58% |
| 710 - 1000 µm | 22% |
| 475 - 710 µm  | 12% |
| 270 - 425 µm  | 8%  |

The acid liquefied starch syrup was pumped through under the same conditions as in example 7. Again the flow rate was controlled in order to keep DE at 62. the half-life was estimated to about 1350 hours and the initial activity to 2.0 g product/h/g particles on dry basis. The composition of the outlet syrup was as follows:

| %DP$_1$ | %DP$_2$ | %DP$_3$ | %DP$_{4+}$ | DE |
|------|------|------|------|----|
| 31 | 34 | 14 | 21 | 62 |

That is slightly less DP$_1$ and a bit more DP$_2$ compared to example 6 at identical DE. The outlet pH was 4.2 - 4.3.

Example 9.

17.5 g wet SAM-10 particles made as described in example 3 was filled in a column and run under the same conditions as earlier. The product syrup at identical DE has a somewhat different composition than seen in the previous examples, that is less $DP_1$ and higher $DP_2$.

| %$DP_1$ | %$DP_2$ | %$DP_3$ | %$DP_4+$ | DE |
|------|------|------|------|----|
| 30 | 37 | 14 | 19 | 62 |

## Example 10

8 g of immobilized neutral α-amylase from __Bacillus__ __subtilis__ (such as that sold under the Trade Mark BAN®) (immobilized as described in example 5) were soaked in a 42 DE acid liquefied starch syrup for two hours. Beforehand pH of the syrup was adjusted to 6.0 and the dry substance content reduced from about 80% to 45%. 100 ppm Kathen 886 was added in order to prevent bacterial contamination. The particles were filled into a water jacketed column with a diameter of 1.5 cm. The column temperature was maintained at 60°C and the liquefied starch syrup was pumped through the enzyme bed. The flow rate was controlled in order to keep the product syrup at a DE of about 62. The initial activity was found to be about 0.7 g product/h/g particles on dry basis. The composition of the outlet syrup is given below:

| %$DP_1$ | %$DP_2$ | %$DP_3$ | %$DP_4+$ | DE |
|------|------|------|------|----|
| 37.2 | 29.3 | 9.8 | 23.6 | 62 |

## Example 11

13 g of immobilized neutral α-amylase from __Bacillus__ __licheniformis__ (such as that sold under the Trade Mark Termamyl®) (immobilized as described in example 5) was tested in the same way as described in the example 10. The composition of the outlet syrup is given below.

| %DP$_1$ | %DP$_2$ | %DP$_3$ | %DP$_4$+ | DE / 62 |
|---|---|---|---|---|
| 35.1 | 28.5 | 14.0 | 22.2 | |

Example 12 (comparison example)

The same substrate as in example 10 was also tested at 60°C with soluble neutral α-amylases such as those sold under the Trade Marks BAN® and Termamyl®, respectively.

The results were as follows:

| | %DP$_1$ | %DP$_2$ | %DP$_3$ | %DP$_4$+ | DE | Dosage[1] | Reaction time |
|---|---|---|---|---|---|---|---|
| BAN, soluble | 34.8 | 33.4 | 10.7 | 21.1 | ~~64.6~~ 63 | 16 KNU | 72 hours |
| BAN, immobil. | 37.2 | 29.3 | 9.8 | 23.6 | ~~65.6~~ 63 | – | – |
| Termamyl, soluble | 32.4 | 29.7 | 15.3 | 22.8 | ~~62.6~~ 61 | 8 KNU | 96 hours |
| Termamyl, immobil. | 35.1 | 28.5 | 14.0 | 22.2 | ~~63.9~~ 62 | | |

1) per g dry substance

It appears from the above that the soluble form of the neutral α-amylases gives rise to a too low content of glucose to satisfy the more rigid specifications for high conversion syrups.

Furthermore, the extremely high dosage of the neutral α-amylases and prolonged reaction time necessary for obtaining a high degree of hydrolysis are completely unsuitable for industrial production.

The features disclosed in the foregoing description, in the following claims and/or in the accompanying drawings may, both separately and in any combination thereof, be material for realising the invention in diverse forms thereof.

CLAIMS

1.    A method for preparing high conversion syrups
which comprises hydrolyzing a liquefied starch by contact
thereof with an alpha-amylase covalently immobilized inside
of a carrier substance said contact being at a temperature
in the range of 55 - 70°C and at a pH of 3 - 7, the alpha-
amylase being sufficiently stable under these conditions to
perform the hydrolysis.

2.    A method according to claim 1 wherein the
hydrolysis is conducted at a pH of 3 - 5.

3.    A method according to claim 1, wherein the
liquefied starch has a DE of 12 - 55.

4.    A method according to claim 1, wherein the
liquefied starch has a DE of 30 - 55.

5.    A method according to claim 1, wherein the
alpha-amylase in the carrier was immobilized from an alpha-
amylase preparation of an acid stable _Aspergillus_ derivable
alpha-amylase, the preparation being essentially free of
transferase and glucoamylase activity.

6.    . A method according claim 5, wherein the acid
stable alpha-amylase is derivable from _Aspergillus_ _niger_.

7.    A method according to claim 6, wherein the
acid stable alpha-amylase is derivable from _Aspergillus_
_niger_ strain DSM 2761.

8.    A method according to claim 1, wherein the
alpha-amylase is a _Bacillus_ derivable alpha-amylase

9.    A method according to claim 8, wherein the
alpha-amylase is a _Bacillus_ _subtilis_ derivable alpha-
amylase.

10.   A method according to claim 8, wherein the
alpha-amylase is a _Bacillus_ _licheniformis_ derivable alpha-
amylase.

11.   An immobilized alpha-amylase product
comprising an alpha-amylase covalently immobilized inside of
a carrier substance, the product being suitable for use in a method for preparing high conversion syrups according to claim 1.

12. An immobilized alpha-amylase product comprising an acid stable *Aspergillus* derivable alpha-amylase covalently immobilized inside of a carrier substance and being essentially free of transferase and glucoamylase activity.

13. An immobilized alpha-amylase product according to claim 12 wherein the acid stable alpha-amylase is derivable from *Aspergillus niger*.

14. An immobilized alpha-amylase product according to claim 13, wherein the acid stable alpha-amylase is derivable from *Aspergillus niger* strain DSM 2761.

1/5

FIG. 1
pH = 4.5

RELATIVE ACTIVITY %

FIG. 2
T = 37 °C

0157638

3|5

FIG. 3
T = 60 °C

RELATIVE ACTIVITY %

FIG. 4
T= 60°

RELATIVE
ACTIVITY
%

○ SAM 14

● SAM 16

FIG.5
pH = 4.5